# EUROPEAN PATENT APPLICATION

(11) **EP 2 399 563 A2**
(43) Date of publication of application: **28.12.2011**
(21) Application number: 10743930.9
(22) Date of filing: 17.02.2010
(51) Int. Cl.: A61H 7/00, A61M 1/08, A61N 5/06

(54) **MASSAGE APPARATUS, AND MASSAGE CUP WITH A DUAL STRUCTURE FOR THE MASSAGE APPARATUS**

(30) Priority: 18.02.2009 KR 20090013408
(71) Applicant: Chang, Tae-Soun, Seoul 120-090 (KR)
(72) Inventor: IM, Hyo-Bin, Gyunggi-do 435-010 (KR)
(74) Representative: Goddar, Heinz J.
(86) International application number: PCT/KR2010/000987
(87) International publication number: WO 2010/095855

(57) **Abstract**

The present invention relates to a massage cup with a dual structure for a massage apparatus, wherein the massage cup is stepped to prevent a vacuum from being easily broken even when the massage cup moves along a curved part of a body, and wherein the massage cup with the dual structure is formed into a single body to enable easy manufacture and to eliminate the necessity of disassembly and assembly, thus enabling the proper washing and disinfection of the massage cup. The massage cup with a dual structure according to the present invention comprises: a cylindrical main body which has a nipple in communication with a negative pressure source via a connection tube, and an open bottom; and a plurality of skin contact portions which extend in the circumferential direction from the bottom of the main body and which are formed into at least one closed loop, wherein the interiors of said skin contact portions are set higher than the exteriors thereof.

## Description

### Technical Field

The present invention relates to a massage apparatus and a massage cup with a dual structure for the massage apparatus, wherein the massage cup is stepped to prevent a vacuum from being easily broken even when the massage cup moves along a curved part of a body, and wherein the massage cup with the dual structure is formed into a single body to enable easy manufacture and to eliminate the necessity of disassembly and assembly, thus enabling proper washing and disinfection of the massage cup.

### Background Art

Generally, a treatment method using a cupping apparatus refers to an oriental medicine treatment method used for vacuum lifeblood treatment, blood purification and improvement of physical constitution. In the treatment method using the cupping apparatus, a cupping cup is put upside down on the affected part to be treated, and air in the cupping cup is drawn out through an inhalation apparatus for taking air to make a vacuum state so that a part of the skin to be treated is inhaled into the interiors of the cupping cup, the muscle is released and nerves are stabilized, pores of the skin are opened, a bodily waste that exits in capillaries of a human body, other toxin, or the like are drawn out through the pores, blood flows smoothly through blood vessels in the body and a clot of blood, pus, or the like is extracted.

The treatment method using the cupping apparatus can be largely classified into two types. One is a treatment method by which a predetermined amount of combustion material is sprayed into an interior of a cupping cup and is combusted for a predetermined amount of time to make the interior of the cupping cup in a vacuum state and to attach the cupping cup to the human body, and the other one is a treatment method by which air in the cupping cup is drawn out by using an inhaler or an inhalation pump to make the interior of the cupping cup in a vacuum state and to attach the cupping cup to the human body.

In cupping apparatuses according to the related art, a cupping cup is provided and is connected to a separate vacuum inhalation apparatus and contacts the skin of the human body to operate the vacuum inhalation apparatus and to reduce pressure in the cupping apparatus, or only the cupping cup is used and contacts the skin of the human body and then moxibustion is performed in the cupping cup or a manual pump is installed at the cupping cup, air in the cupping cup is drawn out in a manual manner by using the manual pump to change the interior of the cupping cup into a vacuum (low pressure) state, thereby performing a cupping procedure on the human body.

However, in such cupping apparatuses, a simple cupping procedure function by which bodily waste or blood is drawn out of the body by simply using a negative pressure, is performed, and it is not easy to adjust an inhalation pressure and thus, the skin tissue may be pressurized, and it is not easy to use the cupping apparatus for skin care.

In order to solve the problems, Korean Utility Model No. 317763 discloses a skin massage apparatus in which a single cylindrical cupping cup is connected to a main body for generating an air pressure, a change of pressure including a positive pressure and a negative pressure in the cupping cup is adjusted in various ways and the number of adjusting the pressure including the positive pressure and the negative pressure and time required for the adjustment are set in advance according to modes.

However, in skin massage apparatuses using cupping according to the related art, it is not easy to perform a massage procedure while the cupping cup moves along several parts of a body curved with various curvatures.

In addition, Korean Utility Model No. 313775 discloses a cupping apparatus having a dual compression plate so as to increase a compression force. However, when the cupping apparatus moves so as to perform a massage procedure, a set negative pressure may be broken. In detail, the foregoing Korean Utility Model No. 313775 has a structure in which the negative pressure cannot be continuously applied to the skin when a massage procedure is performed by moving the cupping apparatus.

Furthermore, Korean Utility Model No. 191615 discloses a structure in which a plurality of ring-shaped, small protrusions are formed into a single body at a lower end of a container so as to reduce oppressive pain that occurs when a strong negative pressure is applied to a cupping apparatus and the affected part is inhaled into the container. However, when a massage procedure is performed by moving a cupping apparatus along a curved part such as a face part, the plurality of ring-shaped, small protrusions disposed at a fine distance and set at the same level cannot easily correspond to the curved face part and thus, a vacuum state may be easily broken.

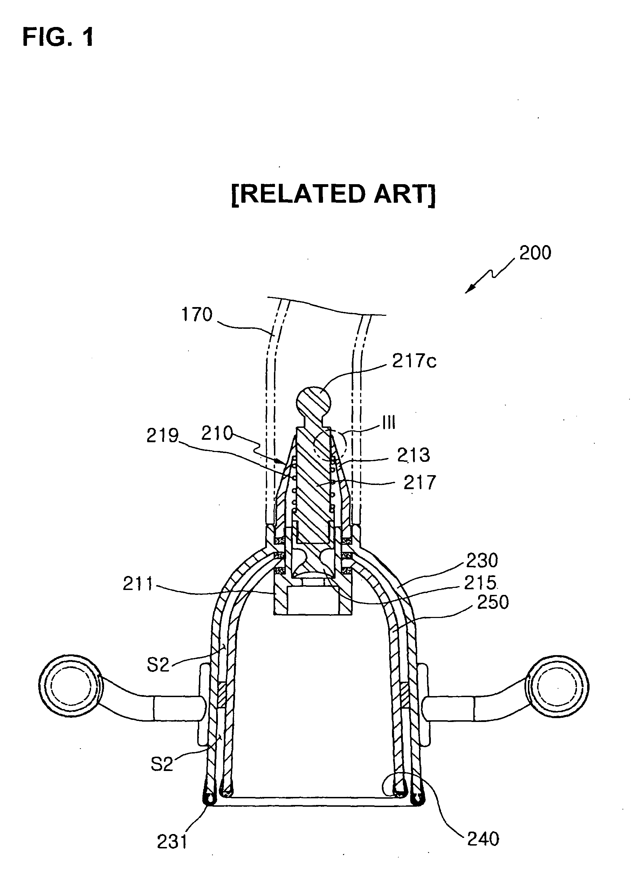

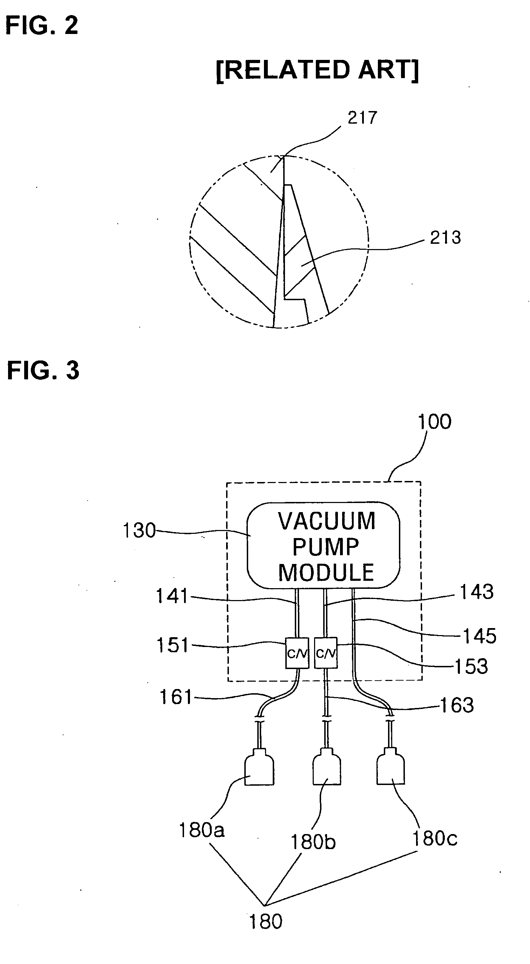

In consideration of the problems of the related art, the present applicant has suggested a skin massage apparatus for removing skin waste by using a negative pressure with a dual cup structure disclosed in Korean Patent No. 856761. The foregoing Korean Patent No. 856761 discloses a massage apparatus that may be applied to various parts of the body by using a dual cupping cup, which will be described with reference to FIGS. 1 and 2.

FIG. 1 is a cross-sectional view of a dual cupping cup according to the related art, and FIG. 2 is an enlarged view of portion III of FIG. 1.

Referring to FIG. 1, a cupping cup 200 includes a valve unit 210 having one side connected to a vacuum pump (not shown) via a connection tube 170, an outer cup 230 and an inner cup 250 each having a top end in communication with the other side of the valve unit 210 and installed to overlap each other while maintaining a gap S2 therebetween.

The valve unit 210 includes a valve housing 211, a cap 213, a connection valve 215, an actuating stem 217, and a return spring 219.

The skin massage apparatus disclosed in Korean Patent No. 856761 uses the single cupping cup 200. When the cupping cup 200 is used, a pressure selection switch of a vacuum inhalation apparatus is manipulated to select a desired negative pressure suitable for a massage part of a body so that the cupping cup 200 moves along the skin to be massaged. In this regard, the cupping cup 200 of the foregoing Korean Patent No. 856761 has a dual cup structure, and first and second packings 231 and 240 formed of a soft material and disposed on a lower end of the cupping cup 200 closely contact the skin and thus, a vacuum state is not easily broken.

In addition, according to the foregoing Korean Patent No. 856761, by removing a negative pressure of the vacuum inhalation apparatus with respect to the cupping cup 200, the connection valve 215 returns to the return spring 219 to maintain a negative pressure state. Furthermore, by separating the connection tube 170 from the cap 213 and by pulling a gripping portion 217c, airtight states of interiors of the inner cup 250 and the outer cup 230 are sequentially released and the inner cup 250 and the outer cup 230 are separated from the skin.

However, the cupping cup 200 includes the valve unit 210 having a complicated structure disposed on an upper portion of the cupping cup 200, and an upper portion of the outer/inner cup 230 or 250 is combined with an outer circumferential portion of the valve unit 210, and a spacer for maintaining the gap S2 between the outer and inner cups 230 and 250 is inserted between the outer and inner cups 230 and 250. Thus, an assembly process is not simple, and a plurality of components are required to configure the cupping cup 200 so that durability of the cupping cup 200 is lowered and a manufacturing cost thereof increases.

In addition, the foregoing Korean Patent No. 856761 is suitable for removing skin waste but cannot achieve acupressure treatment effects for blood circulation etc.

Furthermore, it is desirable to re-use the dual cupping cup 200 after undergoing washing and disinfection. However, the dual cupping cup 200 includes a valve disposed thereon. Thus, the dual cupping cup 200 has a complicated structure, and it is difficult to perform disassembly and assembly of the cupping cup 200, and washing and disinfection cannot be fully performed.

Korean Patent No. 461469 discloses a heating cupping apparatus having a heating unit. In particular, Korean Patent No. 841019 discloses a heating cupping apparatus and a cupping apparatus using the same.

The foregoing Korean Patent No. 461469 suggests a technology for heating air in a cupping apparatus by installing a wire-shaped heating unit at an inner circumferential portion of the cupping apparatus, and the foregoing Korean Patent No. 841019 suggests a technology for transferring heat to an interior of A cupping apparatus by installing a heater at an exterior of the cupping apparatus. However, according to the foregoing Korean Patent No. 461469, a manufacturing process is complicated, and a heat wire is attached to an interior of a cupping cup, and thus it is difficult to perform washing or disinfection of the cupping cup, and durability of the cupping cup is lowered. According to Korean Patent No. 841019, heat is not efficiently transferred to the interior of the cupping apparatus.

The above-suggested related art cupping cup is a dual cupping cup that has a complicated structure and that may not be easily washed and manufactured with a low price, or a cupping cup having a single cup structure that may remove only bodily waste. A cupping cup having a dual structure that is simple and is stepped, or a cupping cup that may remove bodily waste and simultaneously may achieve acupressure treatment effects, has not been suggested.

### Disclosure of the Invention

The present invention provides a massage apparatus and a massage cup with a dual structure for the massage apparatus, wherein the massage cup is stepped to prevent a vacuum from being easily broken even when the massage cup moves along a curved part of a body, and wherein the massage cup with the dual structure is formed into a single body to enable easy manufacture and to eliminate the necessity of disassembly and assembly, thus enabling proper washing and disinfection of the massage cup.

The present invention also provides a massage apparatus and a massage cup with a dual structure for the massage apparatus, wherein the massage cup includes at least one closed loop-shaped skin contact portion and a plurality of skin contact portions having the shape of a plurality of protrusions that may provide acupressure treatment effects to interiors or exteriors of the skin contact portions.

The present invention also provides a message cup including a grapping portion, wherein the massage cup moves along a body in a state where the massage cup is gripped stably by using the grapping portion.

### Technical Solution

According to an aspect of the present invention, there is provided a massage cup with a dual structure for a massage apparatus, the massage cup including: a cylindrical main body comprising a nipple in communication with a negative pressure source via a connection tube, and an open bottom; a plurality of skin contact portions extending in a circumferential direction from the bottom of the cylindrical main body and formed into at least one closed loop, wherein interiors of the plurality of skin contact portions are set higher than exteriors of the skin contact portions.

Each of the plurality of skin contact portions may have a closed loop shape.

At least one of the plurality of skin contact portions may have a closed loop shape, and the remaining skin contact portions may include a plurality of hemispheric protrusions disposed in the circumferential direction at a predetermined gap.

At least one of the plurality of skin contact portions may have a closed loop shape, and the remaining skin contact portions may have a closed loop shape having an open portion.

The plurality of skin contact portions may have one selected from the group consisting of a circular shape, a triangular shape, a rectangular shape, a polygonal shape, and an oval shape.

At least one of the plurality of skin contact portions may have a closed loop shape, and the remaining skin contact portions have a radial pattern.

The skin contact portions having a closed loop shape may have one selected from the group consisting of a polygonal shape, an oval shape, and a circular shape.

The cylindrical main body may further include a gripping portion.

The nipple may be formed on a top or a lateral side of the cylindrical main body.

The massage cup may further include a heating element insert-molded in an interior of the cylindrical main body.

The massage cup may further include an infrared or far-infrared light emitting diode (LED) installed at the top of the cylindrical main body and emitting an infrared or far-infrared light to an interior of the massage cup.

According to another aspect of the present invention, there is provided a massage apparatus including: a vacuum pump module comprising a vacuum pump and a manifold; a negative pressure source comprising a plurality of check valves connected to the manifold; and a plurality of massage cups to which negative pressures are applied from the negative pressure source.

The vacuum pump module may be connected to the plurality of check valves via a plurality of connection tubes or may be connected directly to the massage cups via a connection tube.

The check valves may be connected to the massage cups via connection tubes.

### Brief Description of the Drawings

FIG. 1 is a cross-sectional view of a cupping cup according to the related art;
FIG. 2 is an enlarged view of portion III of FIG. 1;
FIG. 3 is a schematic block diagram of a massage apparatus including a massage cup with a dual structure for the massage apparatus, according to an embodiment of the present invention;
FIG. 4 is a cross-sectional view of a massage cup with a dual structure for a massage apparatus, according to an embodiment of the present invention;
FIGS. 5A and 5B are bottom views of a structure of skin contact portions that may be adopted in the massage cup illustrated in FIG. 4;
FIGS. 6 and 7 are a cross-sectional view and a bottom view of a massage cup with a dual structure for a massage apparatus, according to another embodiment of the present invention;
FIGS. 8 and 9 are a cross-sectional view and a bottom view of a massage cup with a dual structure for a massage apparatus, according to another embodiment of the present invention;
FIGS. 10 and 11 are a cross-sectional view and a bottom view of a massage cup with a dual structure for a massage apparatus, according to another embodiment of the present invention;
FIG. 12 is a cross-sectional view of a massage cup with a dual structure for a massage apparatus, according to another embodiment of the present invention;
FIGS. 13A and 13B are bottom views of a structure of skin contact portions that may be adopted in the massage cup illustrated in FIG. 12;
FIGS. 14A through 14C are bottom views of skin contact portions that may be adopted in the massage cup illustrated in FIG. 4 or FIGS. 6 and 7;
FIGS. 15A through 15E are bottom views of skin contact portions that may be adopted in the massage cup illustrated in FIG. 4 or FIGS. 6 and 7;
FIG. 16 is a cross-sectional view of a modified example in which a gripping portion is added to the massage cup of FIGS. 6 and 7;
FIGS. 17A through 17E are plan views of a gripping portion that may be adopted in the modified example of FIG. 16;
FIG. 18 is a cross-sectional view of a modified example in which a gripping portion is added to the massage cup of FIGS. 8 and 9 and a position of a nipple is changed;
FIGS. 19A through 19E are plan views of a gripping portion and a nipple that may be adopted in the modified example of FIG. 18;
FIGS. 20A through 20D are cross-sectional views of skin contact portions that may be adopted in the massage cups illustrated in FIGS. 4 through 12; and
FIGS. 21 and 22 are cross-sectional views of a massage cup with a dual structure for a massage apparatus, according to another embodiment of the present invention.

### Best mode for carrying out the Invention

The present invention will now be described more fully with reference to the accompanying drawings, in which exemplary embodiments of the invention are shown.

FIG. 3 is a schematic block diagram of a massage apparatus including a massage cup with a dual structure for the massage apparatus, according to an embodiment of the present invention.

Referring to FIG. 3, the massage apparatus includes a negative pressure source 100 and a plurality of massage cups 180 including 180a, 180b, and 180c to which a negative pressure is applied from the negative pressure source 100.

The negative pressure source 100 includes a vacuum pump module 130 including a vacuum pump (not shown) and a manifold (not shown) and a plurality of check valves 151 and 153 connected to the manifold (not shown). When power is applied to the vacuum pump module 130 and a motor installed at the vacuum pump (not shown) is driven, a negative pressure is set to -40 kpa, for example. In this regard, the size of the negative pressure for the vacuum pump is set to be the same as that of a negative pressure of a part that requires the largest negative pressure, from among parts of a body to be massaged.

The vacuum pump module 130 is connected to the plurality of check valves 151 and 153 via a plurality of connection tubes 141 and 143 or is connected directly to the massage cup 180c via a connection tube 145. In this regard, each of the check valves 151 and 153 is connected to each of the massage cups 180a and 180b via connection tubes 161 and 163.

Each of the check valves 151 and 153 may be a check valve having the same structure or function disclosed in Korean Patent Application No. 2009-0005725 or 2009-0005726 (filed on January 22, 2009) that has been filed by the present applicant.

Here, the negative pressure source 100 may be a well-known negative pressure source that operates manually, as well as an electric apparatus that includes a vacuum pump and generates a vacuum pressure by using electricity.

The vacuum pump module 130 transfers a negative pressure that is differently set according to parts of the skin to be massaged to the massage cups 180a and 180b via each of the check valves 151 and 153. When the largest negative pressure, for example, -40 kpa is applied to parts of the skin, the vacuum pump module 130 transfers the negative pressure that is differently set according to parts of the skin to be massaged directly to the massage cup 180c without using the check valves 151 and 153.

In this regard, the check valves 151 and 153 are set in such a way that pre-set, different negative pressures, for example, -15 kpa, -20 kpa, and -30 kpa, are optionally applied to face, neck, and abdomen in consideration of sensitivity with respect to a negative pressure. The check valves 151 and 153 communicate with the plurality of massage cups 180a and 180b via the connection tubes 161 and 163.

The connection tube 145 having no check valve applies the negative pressure of the vacuum pump module 130 to the massage cup 180c without any change of the negative pressure.

When the massage cups 180a and 180b contact the skin and a negative pressure corresponding to a pre-set negative pressure set to each of the check valves 151 and 153 is formed in each of the massage cups 180a and 180b, the check valves 151 and 153 are closed to block the negative pressure provided from the vacuum pump module 130. Contrary to this, when a vacuum state formed between each of the massage cups 180a and 180b and the skin is removed during a skin massage procedure, the check valves 151 and 153 are opened to provide the negative pressure into each of the massage cups 180a and 180b from the vacuum pump module 130. For example, when the vacuum (negative pressure) state formed between the massage cup 180a and the skin is removed during the skin massage procedure and the check valve 151 is in a atmospheric state, or when a higher pressure than the set negative pressure (-15 kpa) such as -5 kpa is applied to the check valve 151, the check valve 151 is in an open state. After that, when a user attaches a front end portion of the massage cup 180a to the skin and maintains the massage cup 180a in an airtight state, the check valve 151 detects that the pre-set negative pressure of -15 kpa is applied into the massage cup 180a from the vacuum pump module 130, blocks a flow path and maintains the pre-set negative pressure of -15 kpa.

In this way, the massage apparatus according to the present invention may perform a massage procedure by using the massage cups 180a, 180b, and 180c by selecting negative pressures suitable for parts of the skin to be massaged. In addition, the user may perform a massage procedure to persons simultaneously by using the massage cups 180a, 180b, and 180c.

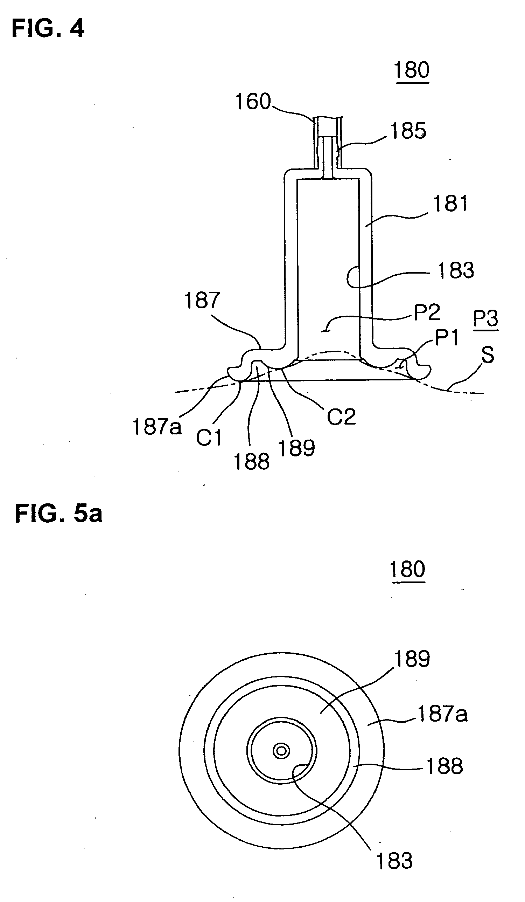

The massage cups 180a, 180b, and 180c may be configured according to embodiments of the present invention illustrated in FIGS. 4 through 12. In this regard, the massage cups 180a, 180b, and 180c are configured to have interiors set higher than exteriors thereof so that a lower end portion of each of the massage cups 180a, 180b, and 180c closely contacts a curved part of the body, and are formed into a single body.

In addition, each of the massage cups 180a, 180b, and 180c according to the present invention include at least one closed loop-shaped skin contact portion and a plurality of skin contact portions having the shape of a plurality of protrusions that may provide acupressure treatment effects to interiors or exteriors of the skin contact portions.

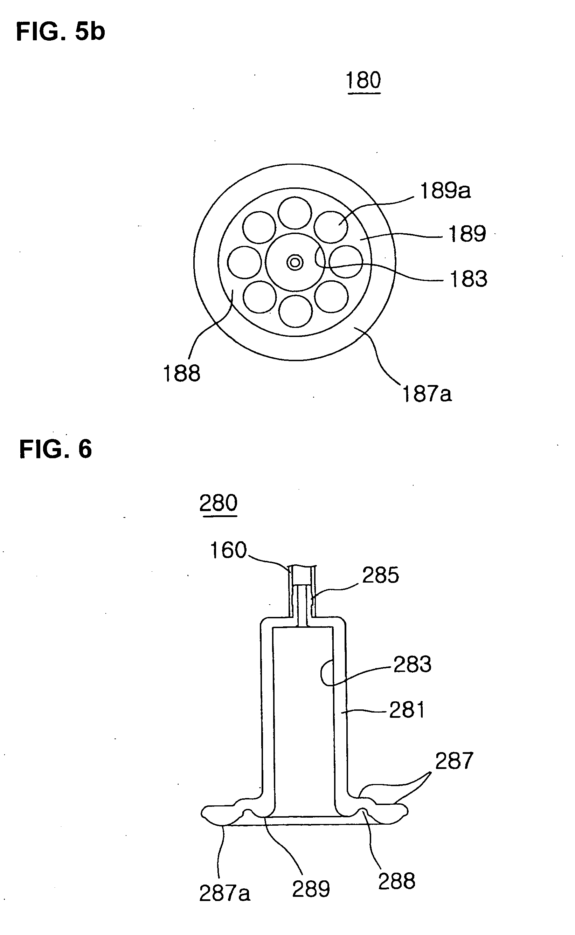

FIG. 4 is a cross-sectional view of a massage cup with a dual structure for a massage apparatus, according to an embodiment of the present invention, and FIGS. 5A and 5B are bottom views of a structure of skin contact portions that may be adopted in the massage cup illustrated in FIG. 4.

Referring to FIG. 4, a massage cup 180 according to the current embodiment of the present invention includes a cylindrical main body 181 having an open bottom and a space portion 183 formed therein, a nipple 185 that protrudes toward an upper portion of the cylindrical main body 181, communicates with the space portion 183 and is connected to the negative pressure source (see 100 of FIG. 3) via a connection tube 160, and an extension portion 187 that is disposed on a lower portion of the cylindrical main body 181 to have a larger size than a diameter of the cylindrical main body 181 and is shaped approximately as a skirt.

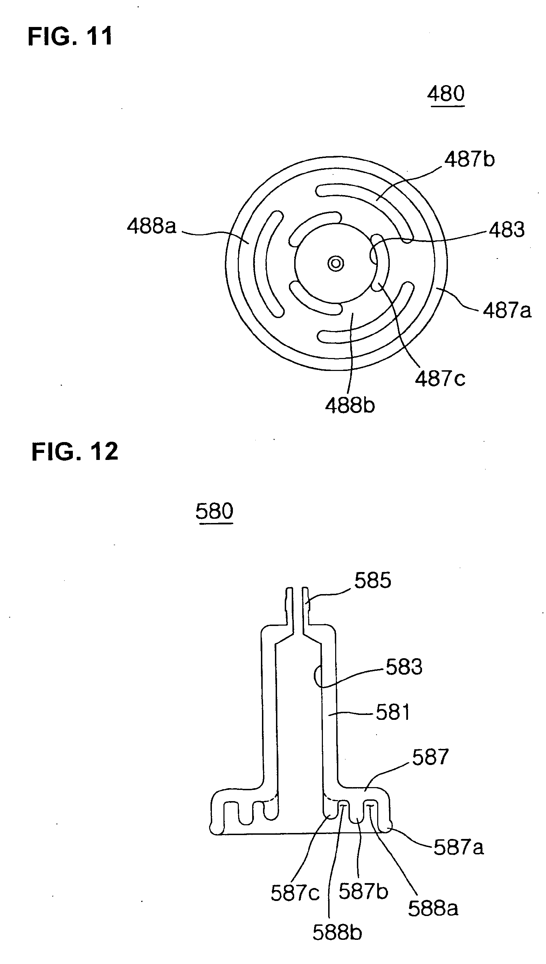

The extension portion 187 includes first and second skin contact portions 187a and 189 stepped to different heights. The first skin contact portion 187a is disposed on a bottom end of the extension portion 187 and is shaped as a closed loop, as illustrated in the bottom view of FIG. 5A. The second skin contact portion 189 is disposed to be separated from the first skin contact portion 187a by a predetermined gap so that a space portion 188 is formed between the second skin contact portion 189 and the first skin contact portion 187a. The second skin contact portion 189 is shaped as a closed loop like in the first skin contact portion 187a or a closed loop having an open portion, similarly to FIG. 11 that will be described later.

The second skin contact portion 189 on the bottom end of the extension portion 187 is shaped as a plurality of hemispheric protrusions 189a disposed to be separated from each other by a predetermined gap in a circumferential direction, as illustrated in the bottom view of FIG. 5B.

As illustrated in the bottom views of FIGS. 5A and 5B, the second skin contact portion 189 may have a closed loop shape or a plurality of hemispheric protrusions and is formed to have the space portion 188 between the second skin contact portion 189 and the first skin contact portion 187a.

In this regard, the first and second skin contact portions 187a and 189 naturally contact a curved part of the body and have a predetermined curvature so that the skin may not be injured when a massage procedure is performed along the skin.

In addition, the second skin contact portion 189 protrudes in approximately a downward direction of the massage cup 180, like in the first skin contact portion 187a. In this regard, as the first skin contact portion 187a protrudes to a larger length than the second skin contact portion 189, a stepped portion is formed between the first and second skin contact portions 187a and 189.

Thus, when the massage cup 180 contacts the skin S and a predetermined negative pressure is applied to an interior 183 of the massage cup 180 from the negative pressure source 100 via the connection tube 160, the first and second skin contact portions 187a and 189 are sequentially inhaled into the skin S. In this regard, the skin S in a portion C1 that contacts the first skin contact portion 187a is further expanded, i.e., pressurized and stimulated than the skin S in a portion C2 that contacts the second skin contact portion 189. Furthermore, a first negative pressure P1 that is lower than an atmospheric pressure P3 is formed in the space portion 188 between the first and second skin contact portions 187a and 189, and a second negative pressure P2 that is the same as a set negative pressure of the check valve 151 or 153 of FIG. 3 is formed in the interior 183 of the cylindrical main body 181. In other words, the second negative pressure P2 is set to be lower than the first negative pressure P1. Thus, since the second negative pressure P2 is set to be lower than the first negative pressure P1 (high degree of vacuum), a part of the skin to be massaged located in the interior 183 of the cylindrical main body 181 is expanded with a stronger inhalation force than in a part of the skin to be massaged located in the space portion 188.

In this state, a massage procedure is performed while the massage cup 180 moves along the part of the body to be massaged. In this regard, when air flows between the first skin contact portion 187a and the skin, the first negative pressure P1 of the space portion 188 is released, and the space portion 188 is in an atmospheric pressure. However, the massage procedure may be performed in a state where the interior 183 of the cylindrical main body 181 is not affected by a change of pressure of the first negative pressure P1 and is maintained at the second negative pressure P2.

After that, during the massage procedure, air flows into the interior 183 of the cylindrical main body 181 and the second negative pressure P2 is released, and the interior 183 of the cylindrical main body 181 is in an atmospheric pressure, and the check valve 151 or 153 of FIG. 3 is set to open the flow path. After that, when the user allows a dual massage tool to contact the skin again, the check valve 151 or 153 of FIG. 3 re-operates so that a negative pressure is supplied to the interior 183 of the massage cup 180 and the negative pressure defined in the check valve 151 or 153 of FIG. 3 is formed in the interior 183 of the massage cup 180. In this way, the negative pressure may be maintained in the interior 183 of the massage cup 180 by operating the check valve 151 or 153 of FIG. 3 while the massage procedure is performed.

In FIG. 4, when the second skin contact portion 189 includes a plurality of hemispheric protrusions 189a, the space portion 188 and the interior 183 of the cylindrical main body 181 are set to have the same negative pressure. Thus, relatively strong stimulation (pressure) is applied to a plurality of parts of the skin to be massaged that contact the plurality of hemispheric protrusions 189a, compared to the remaining parts of the skin located in the massage cup 180.

In addition, when the user slides the massage cup 180 on an adjacent part of the skin to be massaged in this state, the plurality of parts of the skin to be massaged that contact the plurality of hemispheric protrusions 189a move and stimulate a fine lymphatic vessel with respect to the parts of the skin to be massaged. As a result, strong stimulation (i.e., acupressure) is applied to the parts of the skin to be massaged so that blood circulation and massage effects are promoted.

Furthermore, when the massage procedure is performed in a state where a strong negative pressure is applied to the palm or sole that is called as a reduced part of the body since many nerves connected to various organs are concentrated in the palm or sole, spots on the body connected to various organs are stimulated, and the organs may be activated.

Hereinafter, a massage cup with a dual structure for a massage apparatus, according to embodiments of the present invention illustrated in FIGS. 6 through 22 will be described. Like elements in those of FIG. 4 perform the same functions and thus, only a modified shape thereof will be described briefly.

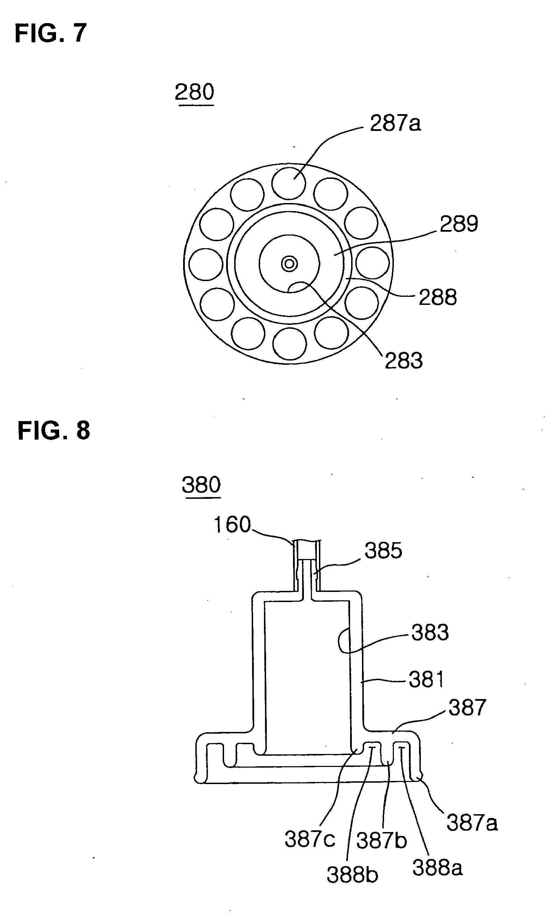

FIGS. 6 and 7 are a cross-sectional view and a bottom view of a massage cup with a dual structure for a massage apparatus, according to another embodiment of the present invention.

Referring to FIGS. 6 and 7, a massage cup 280 according to the current embodiment of the present invention has almost the same configuration as the massage cup 180 of FIG. 4. The only difference is that an extension portion 287 disposed on a bottom end of the massage cup 280 and having a plurality of hemispheric protrusions 287a is set lower than that of the extension 187 of FIG. 4 and thus the depth of a space 288 is not larger than that of the space portion 188 of FIG. 4. Thus, the massage cup 280 of FIGS. 6 and 7 is suitable for performing a massage procedure on a part that is not severely curved, such as back or leg.

Unexplained reference numerals 281, 283, and 285 of FIGS. 6 and 7 represent a cylindrical main body, a space portion of the cylindrical main body, and a nipple, respectively.

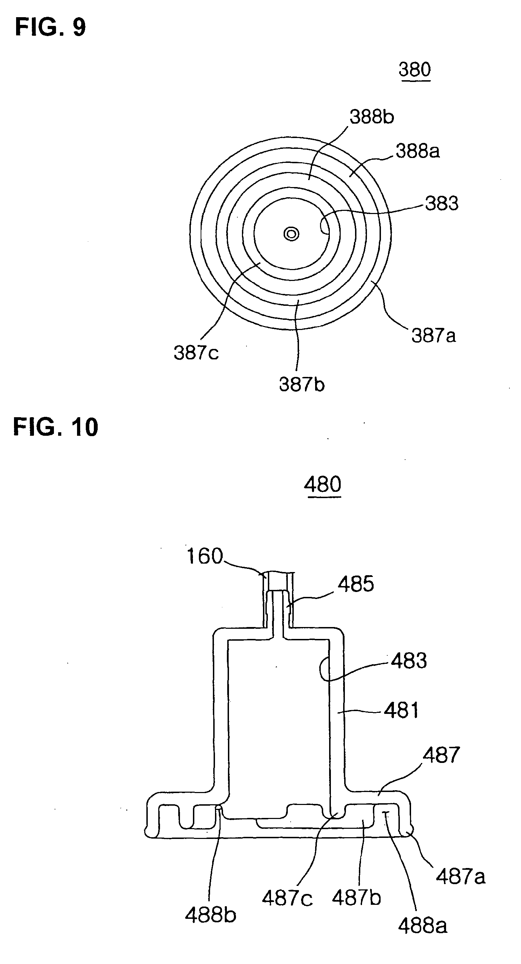

FIGS. 8 and 9 are a cross-sectional view and a bottom view of a massage cup with a dual structure for a massage apparatus, according to another embodiment of the present invention.

Referring to FIGS. 8 and 9, a massage cup 380 according to the current embodiment of the present invention has a higher extension portion 387 than the extension portion 287 of FIGS. 6 and 7 and includes two deep space portions 388a and 388b and first through third skin contact portions 387a, 387b, and 387c having a closed loop shape. The first skin contact portion 387a is disposed in a closed loop shape on the outermost exterior of the massage cup 380, and the space portion 388a is formed in an interior of the first skin contact portion 387a, and the second skin contact portion 387 is formed to have another closed loop shape. Last, the space portion 388b is formed in an interior of the second skin contact portion 387b, and the third skin contact portion 387c is formed.

Here, the first through third skin contact portions 387a, 387b, and 387c may be stepped so as to contact the curved part of the body in such a way that a height of lower ends of the first through third skin contact portions 387a, 387b, and 387c is set to be gradually increased toward interiors of the first through third skin contact portions 387a, 387b, and 387c other than exteriors thereof.

However, the first through third skin contact portions 387a, 387b, and 387c may be configured in such a way that the second and third skin contact portions 387b and 387c have lower ends set at the same level and are set higher than the first skin contact portion 387a or the first and second skin contact portions 387a and 387b have lower ends set at the same level and are set lower than the third skin contact portion 387c.

In addition, the first through third skin contact portions 387a, 387b, and 387c may also be configured in such a way that the first and third skin contact portions 387a and 387c have lower ends set at the same level and are set lower or higher than the second skin contact portion 387b.

Unexplained reference numerals 381, 383, and 385 of FIGS. 8 and 9 represent a cylindrical main body, a space portion of the cylindrical main body, and a nipple, respectively.

FIGS. 10 and 11 are a cross-sectional view and a bottom view of a massage cup with a dual structure for a massage apparatus, according to another embodiment of the present invention.

Referring to FIGS. 10 and 11, a massage cup 480 according to the current embodiment of the present invention has almost the same configuration as the massage cup 380 of FIGS. 8 and 9. The only difference is that second and third skin contact portions 487b and 487c have a closed loop shape having an open portion. Thus, a space may be formed in a closed loop having an open portion as well as space portions 488a and 488b.

Unexplained reference numerals 481, 483, and 485 of FIGS. 10 and 11 represent a cylindrical main body, a space portion of the cylindrical main body, and a nipple, respectively.

The massage cup 480 of FIGS. 10 and 11 have a similar function to that of FIG. 5B, and as a result of performing the function, bodily waste may be removed from the skin and acupressure treatment effects may be achieved.

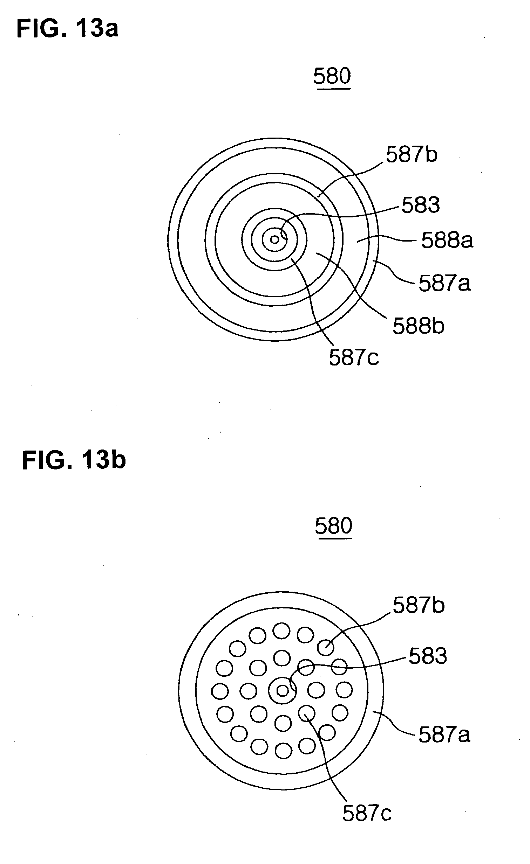

FIG. 12 is a cross-sectional view of a massage cup with a dual structure for a massage apparatus, according to another embodiment of the present invention. FIGS. 13A and 13B are bottom views of a structure of skin contact portions that may be adopted in the massage cup illustrated in FIG. 12. Referring to FIG. 12, a massage cup 580 according to the current embodiment of the present invention has almost the same configuration as the massage cup 380 of FIGS. 8 and 9. The only difference is that a space portion 582 is narrow.

Referring to FIG. 13A, a first skin contact portion 587a has a closed loop shape, and a second skin contact portion 587b also has a closed loop shape in a state where a space portion 588a is interposed between the first and second skin contact portions 587a and 587b, and a third skin contact portion 587c also has a closed loop shape in a state where a space portion 588b is interposed between the second and third skin contact portions 587b and 587c.

As illustrated in the bottom view of FIG. 13B, the second and third skin contact portions 587b and 587c may be formed in a shape of a plurality of hemispheric protrusions disposed in a circumferential direction in a state where the space portion 588b is interposed between the second and third skin contact portions 587b and 587c.

As illustrated in the bottom views of FIGS. 13A and 13B described above, the second and third skin contact portions 587b and 587c may have a closed loop shape or a shape of a plurality of hemispheric protrusions and are formed to have the space portion 588b interposed therebetween.

Unexplained reference numerals 581, 583, and 585 of FIG. 12 and FIGS. 13A and 13B represent a cylindrical main body, a space portion of the cylindrical main body, and a nipple, respectively.

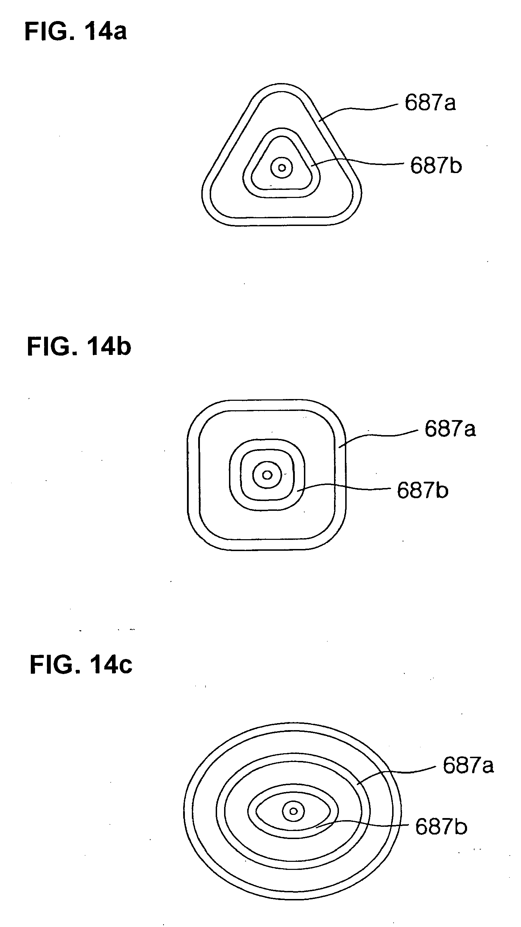

FIGS. 14A through 14C are bottom views of skin contact portions that may be adopted in the massage cup of FIG. 4 or FIGS. 6 and 7.

As illustrated in FIGS. 14A through 14C, first and second skin contact portions 687a and 687b may be shaped as a triangle, a rectangle and an oval as well as a circular ring suggested in the above embodiments,

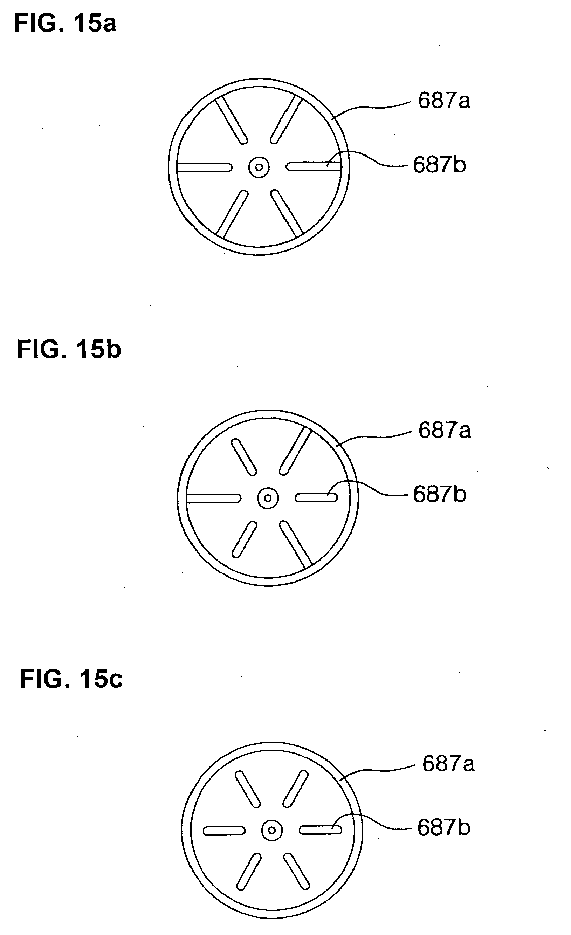

FIGS. 15A through 15E are bottom views of skin contact portions that may be adopted in the massage cup 180 of FIG. 4 or the massage cup 280 of FIGS. 6 and 7.

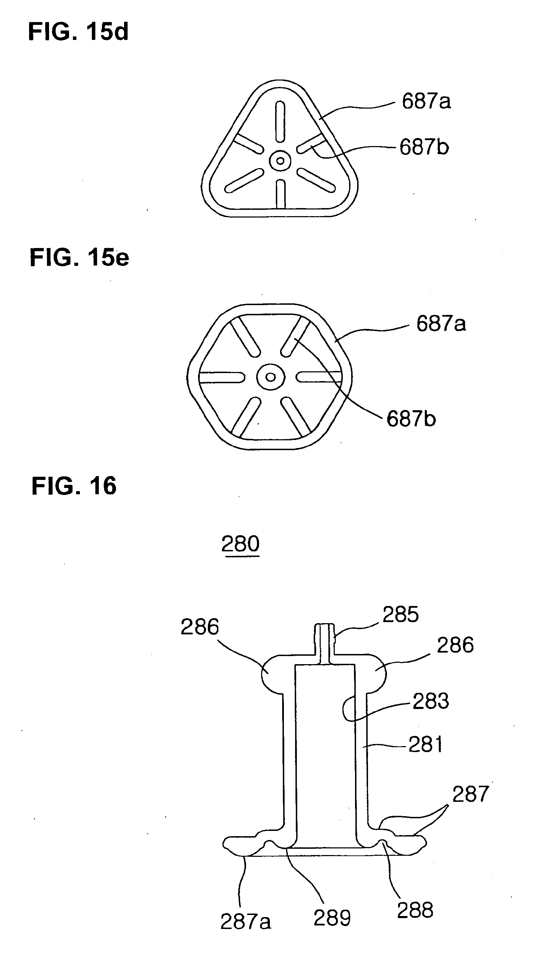

As illustrated in FIGS. 15A through 15C, the first skin contact portion 687a disposed on an exterior of the massage cup 180 of FIG. 4 or the massage cup 280 of FIGS. 6 and 7 may have a circular closed loop shape, and the second skin contact portion 687b disposed on an interior of the massage cup 180 of FIG. 4 or the massage cup 280 of FIGS. 6 and 7 may be formed in a radial pattern having various shapes. In addition, as illustrated in FIGS. 15D and 15E, instead of the circular skin contact portion, the first skin contact portion 687a disposed on the exterior of the massage cup 180 of FIG. 4 or the massage cup 280 of FIGS. 6 and 7 may have a closed loop shape in a polygon such as a triangle, a pentagon, a rectangle, or the like, or an oval, and the second skin contact portion 687b disposed on the interior of the massage cup 180 of FIG. 4 or the massage cup 280 of FIGS. 6 and 7 may be formed in a radial pattern.

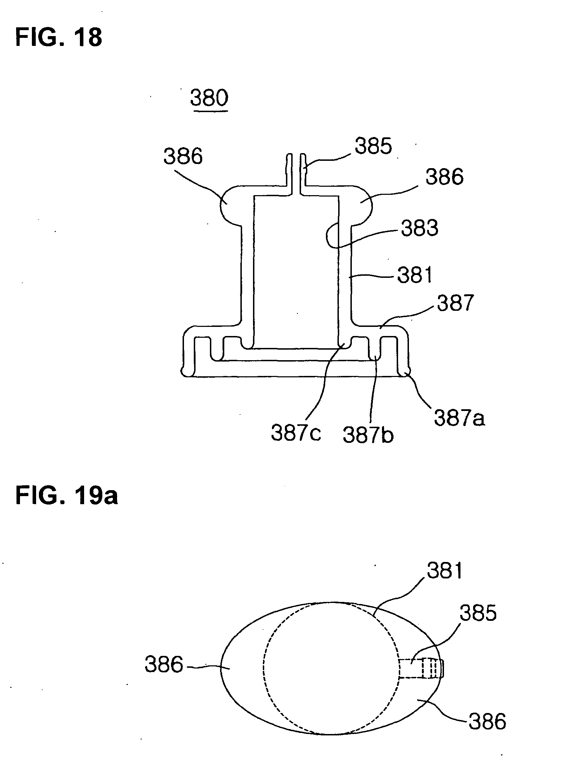

FIG. 16 is a cross-sectional view of a modified example in which a gripping portion is added to the massage cup of FIGS. 6 and 7, and FIGS. 17A through 17E are plan views of a gripping portion that may be adopted in the modified example of FIG. 16. FIG. 18 is a cross-sectional view of a modified example in which a gripping portion is added to the massage cup of FIGS. 8 and 9 and a position of a nipple is changed. The related art massage cup disclosed in Korean Patent No. 856761 suggests the structure in which a ring-shaped bracket is coupled to an outer circumferential portion of the massage cup and a connection bar and a handle are formed at both sides of the ring-shaped bracket. However, in such a structure, a manufacturing process is complicated, and a manufacturing cost increases.

According to the present invention, in consideration of this, a gripping portion 286 is formed in an upper portion of a cylindrical main body 281 into a single body.

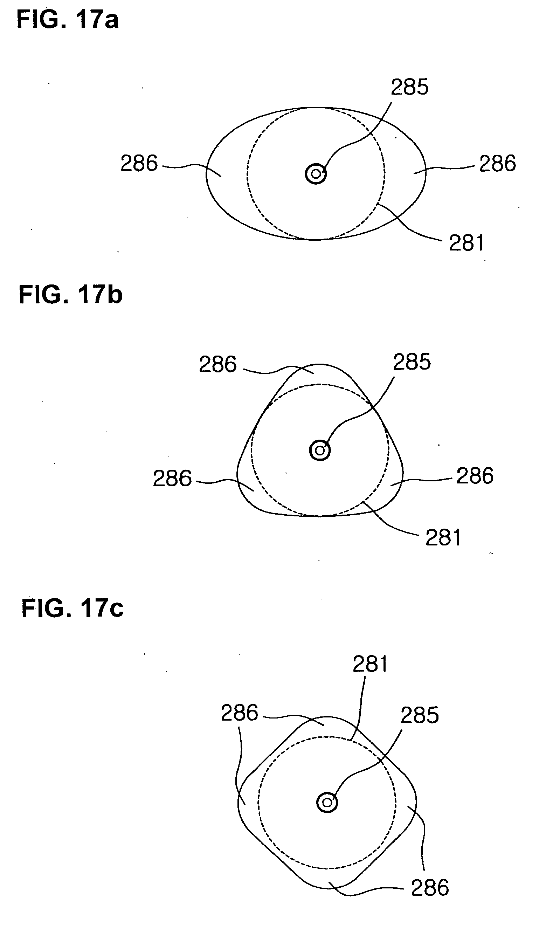

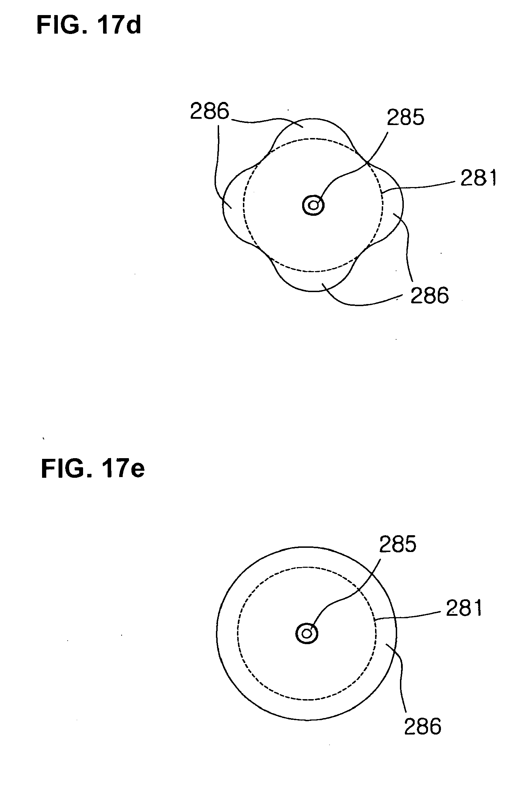

The gripping portion 286 may have an oval shape, a triangular shape, a rectangular shape, a rectangular shape with rounded corners, a circular shape, or the like, as illustrated in FIGS. 17A through 17E and may have any structure that is convenient for the user to grip the massage cup.

In the current embodiment, a nipple 285 having a connection tube coupled thereto is disposed on an upper portion of the cylindrical main body 281. However, as illustrated in FIG. 18, a gripping portion 386 is formed in an upper portion of a cylindrical main body 381, and a position of a nipple 385 disposed on the upper portion of the cylindrical main body 381 is moved to a lateral side of the cylindrical main body 381 so as to improve conveniences when the user grips the massage cup. The massage cup of FIG. 18 uses the structure of FIGS. 8 and 9.

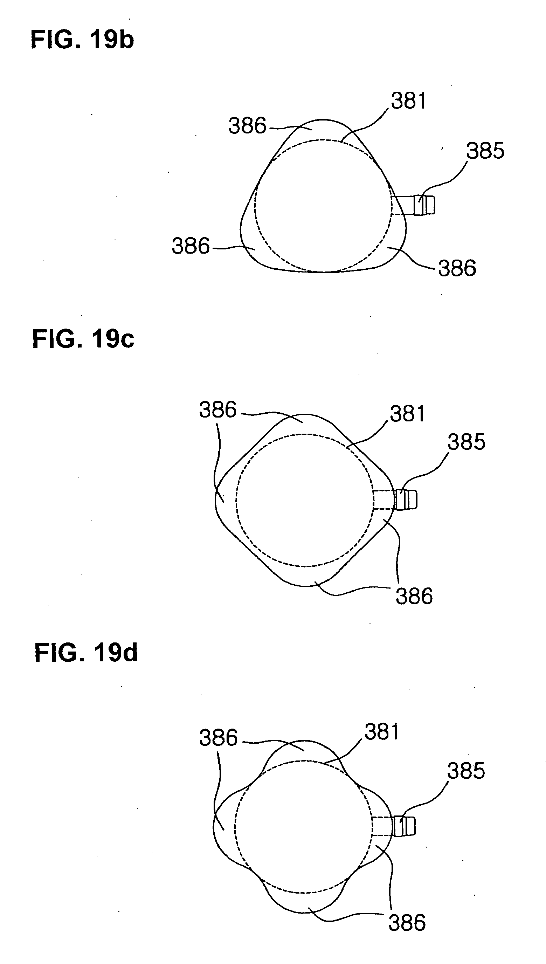

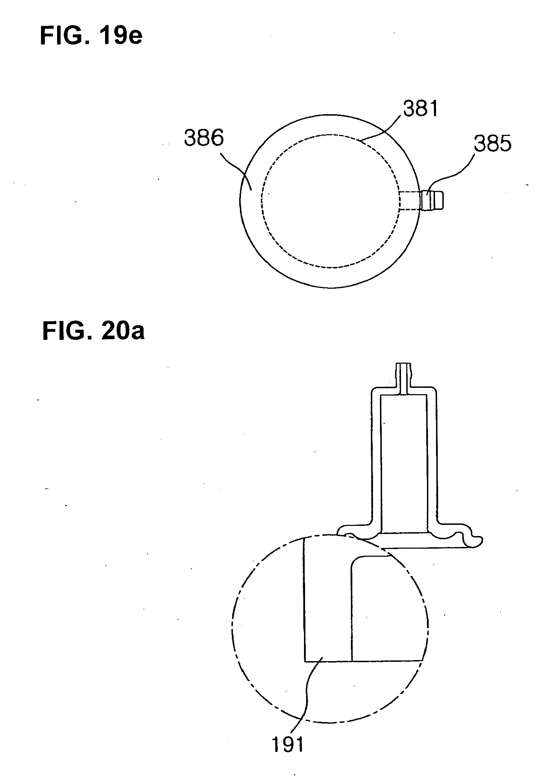

FIGS. 19A through 19E are plan views of a gripping portion and a nipple that may be adopted in the modified example of FIG. 18.

Referring to the plan views of FIGS. 19A through 19E, a gripping portion 386 that may be adopted in the modified example of FIG. 18 may have an oval shape, a triangular shape, a rectangular shape, a rectangular shape with rounded corners, a circular shape, or the like and may have any structure that is convenient for the user to grip the massage cup.

Skin contact portions of the massage cup suggested in the above embodiment have round front end portions but may be modified, as illustrated in FIGS. 20A through 20D.

FIGS. 20A through 20D are cross-sectional views of skin contact portions that may be adopted in the massage cups illustrated in FIGS. 4 through 12.

First, as illustrated in FIG. 20A, when a skin contact portion 191 has a bottom surface perpendicular to an inner circumferential surface and an outer circumferential surface of an interior or exterior of a cylindrical main body, for example, a boundary portion of the skin contact portion 191 that contacts the skin protrudes toward an exterior of the massage cup, may contact the skin in a plane and may be used to increase an intensity of stimulation.

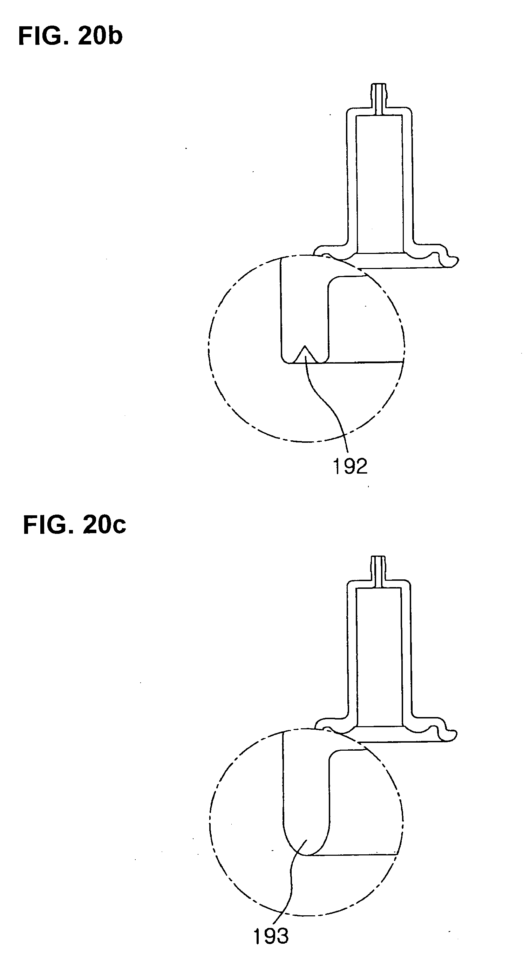

A skin contact portion 192 having a shape illustrated in FIG. 20B has a dual ring shape having a groove. Thus, when the skin contact portion 192 contacts the skin, the user does not feel sore, and a large pressure is applied to the skin, and the skin contact portion 192 may contact the skin easily.

A skin contact portion 193 having a shape illustrated in FIG. 20C has a hemispheric shape, and a boundary portion of the skin contact portion 193 that contacts the skin does not protrude toward an exterior of the massage cup, and a contact area is large, and thus, a large pressure is applied to the skin, and the skin contact portion 193 may contact the skin easily and is efficient to first massage for removing bodily waste and may be used in performing a massage procedure of face and back.

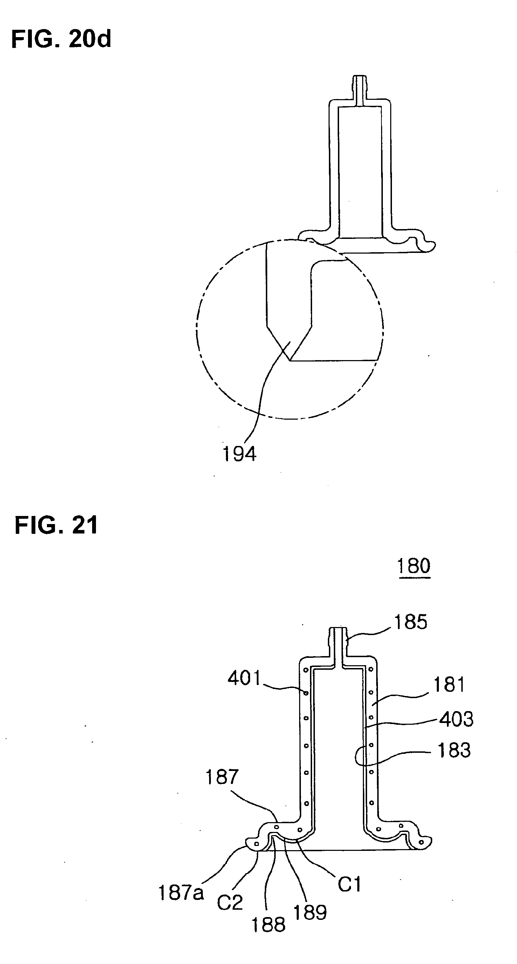

A skin contact portion 194 having a shape illustrated in FIG. 20D has a wedge shape, and a pressure generated when the skin contact portion 194 contacts the skin, is low but stimulation thereof is strong. Thus, the skin contact portion 194 is suitable for achieving acupressure treatment effects on the skin of the palm or sole.

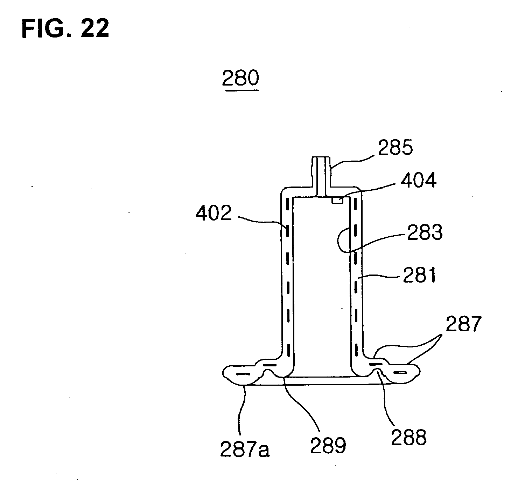

FIGS. 21 and 22 are cross-sectional views of a massage cup with a dual structure for a massage apparatus, according to another embodiment of the present invention.

Referring to FIGS. 21 and 22, the massage cup with a dual structure for a massage apparatus according to the current embodiment of the present invention has the same basic structure as that of FIG. 4. The only difference is that a part of the skin to be massage is heated by using an interior of the massage cup and simultaneously an infrared light or a far-infrared light is emitted from the massage cup.

To this end, first, a nichrome wire heater 401 having a linear shape such as a nichrome wire is used in interiors of a cylindrical main body 181 and an extension portion 187, or a surface-shaped heater 402 formed by slitting an FeCrAl alloy or an amorphous metal thin plate is inserted in the massage cup in an insert molding manner when injection molding is performed.

Power supply to the heaters 401 and 402 may be used by inserting a power plug in a power terminal (not shown) drawn out of an exterior of the cylindrical main body 181 from the heaters 401 and 402, if necessary.

In addition, when the massage cup is heated by the heaters 401 and 402, as illustrated in FIG. 21, a ceramic coating layer 403 coated with a ceramic material for emitting a far-infrared light may be formed in the interiors of the cylindrical main body 181 and the extension portion 187, or when power is supplied to an upper portion of a cylindrical main body 281, as illustrated in FIG. 22, an infrared or far-infrared light emitting diode (LED) 404 for emitting an infrared or far-infrared light may be installed at a top of the cylindrical main body 181.

Thus, when power is supplied to the heaters 401 and 402, air in the massage cup with a dual structure is heated, and temperature of the skin on which a massage procedure is performed, rises. Thus, blood circulation may be smoothly performed, and metabolism may be promoted so that thrombus is solved and the effect of cupping treatment is improved. In addition, an infrared or far-infrared light is emitted from the ceramic coating layer 403 or the infrared or far-infrared LED 404 so that cupping effects may be further improved.

The above embodiment is applied to the massage cup of FIG. 4 but may be applied to the other embodiments in the same way.

In the above embodiment, a first or second skin contact portion includes a plurality of hemispheric protrusions. However, the first or second skin contact portion may have other shapes such as a conic shape, a square shape as well as a hemispheric shape.

A massage apparatus using the massage cup according to the present invention may be used by connecting a plurality of massage cups to which different negative pressures are applied from a negative pressure source according to parts of the skin to be massaged and may be applied by changing a diameter of the massage cup and a shape of a skin contact portion according to parts of the skin to be massage or purposes of use.

While the present invention has been particularly shown and described with reference to exemplary embodiments thereof, it will be understood by those of ordinary skill in the art that various changes in form and details may be made therein without departing from the spirit and scope of the present invention as defined by the following claims.

### Industrial Applicability

According to the present invention, a massage cup with a dual structure having stepped edges is manufactured in a simple structure so as to improve convenience when washing and disinfection of the massage cup is performed, and furthermore, a plurality of hemispheric protrusions are formed in a skin contact portion to remove bodily waste and add an acupressure treatment function, thereby improving efficiency of massage.

## Claims

1. A massage cup with a dual structure for a massage apparatus, the massage cup comprising:
a cylindrical main body comprising a nipple in communication with a negative pressure source via a connection tube, and an open bottom;
a plurality of skin contact portions extending in a circumferential direction from the bottom of the cylindrical main body and formed into at least one closed loop,
wherein interiors of the plurality of skin contact portions are set higher than exteriors of the skin contact portions.

2. The massage cup of claim 1, wherein each of the plurality of skin contact portions has a closed loop shape.

3. The massage cup of claim 1, wherein at least one of the plurality of skin contact portions has a closed loop shape, and the remaining skin contact portions comprise a plurality of hemispheric protrusions disposed in the circumferential direction at a predetermined gap.

4. The massage cup of claim 1, wherein at least one of the plurality of skin contact portions has a closed loop shape, and the remaining skin contact portions have a closed loop shape having an open portion.

5. The massage cup of claim 2, wherein the plurality of skin contact portions have one selected from the group consisting of a circular shape, a triangular shape, a rectangular shape, a polygonal shape, and an oval shape.

6. The massage cup of claim 1, wherein at least one of the plurality of skin contact portions has a closed loop shape, and the remaining skin contact portions have a radial pattern.

7. The massage cup of claim 6, wherein the skin contact portions having a closed loop shape have one selected from the group consisting of a polygonal shape, an oval shape, and a circular shape.

8. The massage cup of claim 1, wherein the cylindrical main body further comprises a gripping portion.

9. The massage cup of claim 1, wherein the nipple is formed on a top or a lateral side of the cylindrical main body.

10. The massage cup of claim 1, further comprising a heating element insert-molded in an interior of the cylindrical main body.

11. The massage cup of claim 1, further comprising an infrared or far-infrared light emitting diode (LED) installed at the top of the cylindrical main body and emitting an infrared or far-infrared light to an interior of the massage cup.

12. A massage apparatus comprising:
a vacuum pump module comprising a vacuum pump and a manifold;
a negative pressure source comprising a plurality of check valves connected to the manifold; and
a plurality of massage cups to which negative pressures are applied from the negative pressure source.

13. The massage apparatus of claim 12, wherein the vacuum pump module is connected to the plurality of check valves via a plurality of connection tubes or is connected directly to the massage cups via a connection tube.

14. The massage apparatus of claim 13, wherein the check valves are connected to the massage cups via connection tubes.
